Europäisches Patentamt

**European Patent Office**

**Office européen des brevets**

(11) Publication number: **0 006 609**
A1

# EUROPEAN PATENT APPLICATION

(21) Application number: **79102122.3**

(22) Date of filing: **26.06.79**

(51) Int. Cl.³: **A 61 F 5/46**

(30) Priority: **04.07.78 IT 2529478**
**04.07.78 IT 2223878**
**06.02.79 IT 1991879**

(43) Date of publication of application: **09.01.80**
**Bulletin 80/1**

(84) Designated Contracting States: **AT BE CH DE FR GB LU NL SE**

(71) Applicant: **Boschetti, Enrica, Via Melchiorre Gioia, I-171-20125 Milano (IT)**

(72) Inventor: **Boschetti, Enrica, Via Melchiorre Gioia, I-171-20125 Milano (IT)**

(74) Representative: **Ferraiolo, Ruggero, Viale Tunisia, 29, I-20124 Milano (IT)**

(54) **Vaginal diaphragm.**

(57) A vaginal diaphragm has a peripheral structure (3') of oval or circular outline which is straddled by an elastomeric cap (2'). In addition, the peripheral structure (3') may be of circular or of oval or elliptical cross-section in direction normal to the major axis of the oval outline, thus providing a relatively flat outer circumferential surface which engages the wall of the vaginal cavity over a comparatively large area and, hence, with low specific surface pressure. The elastomeric cap (2') is joined to the peripheral structure (3') at the level of the mid-plane (M–M) of the diaphragm. The size of the diaphragm can be increased or decreased by bending one or more shape-retaining sections (8') housed in a closed coil spring (4') so that a single diaphragm can be readily accomadated to a number of different size requirements.

0006609

Vaginal diaphragm consisting of a dome made of elastomeric material which is joined to a peripherical closed coil spring housing a bendable shape-retaining armour.

The invention relates to an improved vaginal diaphragm con sisting of a dome made of elastomeric material which is joined to a peripherical closed coil spring housing a bendable shape-retaining armour.

Vaginal diaphragm are known in which the periphery is circu lar, the cross section of the peripherical structure, formed by the peripherical coil spring and by the peripherical edge of the dome, is joined to said peripherical structure tangent with said circular cross section. In some known diaphragms the peripherical coil spring (that in the following will be referred to as "spring" only) houses a bendable shape-retaining armour consisting of metal arched sections which are connected each other by small coil springs so that, to intro duce the diaphragm into the vaginal cavity, the diaphragm may be bent about said small coil springs by lightly pressing two opposite points of the peripherical structure by two fingers. In other known diaphragms no armour is housed in the spring. It is known that the scope of said armour is to assure the engagement of the peripherical structure to the inner wall of the vaginal cavity.

A diaphragm of this type is described in "Population Reports" of Department of Medical and Public Affairs, The George Washington University - Medical Center, 2001 S. Street, N.W. Washington D.C. 20009.

Such known diaphragms do not allow to vary the perimeter which requires to provide for a fairly large variety of diaphragms of different diameters. The circular shape of the diaphragms, the spring of which houses the arched sections, does not fit the nearly oval cross section of the vaginal cavity and this causes non uniform and troubling pressures of the diaphragm on the inner wall of said cavity. On the other hand, with the diaphragms which do not house an armour, the engagement with said inner wall is not enough to assure a safe contraceptive barrier. Moreover, such diaphragms are to be introduced in said cavity with the dome convexity outwardly directed and, consequently, a fingernail could nick or pierce the dome, when extracting the diaphragm, since no good grip point is available. Finally, the circular dome, once introduced in said cavity, becomes approximately oval and takes folds which disturb the uniform distribution of the spermicid substance which is generally used with the diaphragm.

Accordingly, it is an object of the invention to overcome the prior-art disadvantages.

A more particular object of the invention is to provide a va ginal diaphragm of safer and easier use as well as a diaphragms which allows to manufacture a reduced number of different

Dott. Ing. Ruggero Ferraiolo

sizes to meet the requirements of any user.

The invented diaphragm comprises a spring, alternatively of circular or oval cross section, which houses a bendable shape-retaining armour capable of being deformed easily by the fingers so as to adjust the plan shape and dimensions of the diaphragm; the dome is joined to the peripherical structure at the level of the mid-plane of the diaphragm; the plan periphery is, alternatively, circular or oval according to the kind of armour housed in the spring.

The main advantages of the invented diaphragm are set forth herebelow.

An embodiment of the diaphragm provides for the bendable shape-retaining armour within the spring consisting of two arched sections of circular cross section which sections have pointed ends connected by means of two small coil springs; the plan of such a diaphragm is oval shaped and the diaphragm is capable of being extended or shortened with respect to the nominal length of the selected diaphragm which means to adjust the diaphragm to the specific required size. Such an adjustment is made by conveniently bending outwardly or inwardly the central length of the arched sections or the lengths close to the ends thereof. Moreover, the oval shape of the diaphragm well fits the vaginal cavity and therefore causes fairly uniform pressures on the inner wall of said cavity.

Another embodiment of the diaphragm provides for bendable shape-retaining armour within the spring consisting of a circular closed section of flat cross section and of a spring of oval

Dott. Ing. Ruggero Ferraiolo

cross section the major axis of which is perpendicular to the mid-plane of the diaphragm. Then, it will be appreciated that the whole peripherical structure of such diaphragm is of oval cross section and that the supporting surface of said structure on said inner wall is well larger than the supporting surface of the circular cross section. Moreover, the circular closed section housed in the spring is capable of being deformed by the fingers in order to adjust the dimensions of the peripherical structure.

A better engagement of the dome with the cervix is allowed by the dome being joined to the peripherical structure at the level of the mid-plane of the diaphragm since the dome diameter is reduced; this makes the diaphragm safer and also reduces the dome folds which could badly affect the uniform distribution of the spermicide substance. Further, when using such diaphragm it is of no importance to keep outwardly a face or the other due to the plan symmetry of the dome about the mid-plane of the diaphragm. Still more finally, the relief of the peripherical structure from the dome is a good grip point for a finger which extracts the diaphragm from said cavity.

The novel features which are considered as characteristics of the invention are set forth in the appended claims. The construction of the invention as well as additional objects and advantages thereof will however be best understood from the following description of the specific embodiments in connection with the accompanying drawings.

FIG.1 is a top plan,view of a diaphragm embodying the invention;

Dott. Ing. Ruggero Ferraiolo

0006609

FIG. 2 is a section taken on line II-II of FIG.1;

FIG. 2a is a simplified section similar to the one in FIG. 2, but illustrating a prior-art device for comparison;

FIG. 3 is a top plan view similar to the one in FIG. 1, but with portions broken away to facilitate illustration of the interior construction of the device;

FIG. 4 is a perspective view of the device in preceeding FIGURES, showing it bent in preparation for its introduction into the vaginal cavity;

FIG. 5 is a plan view of the device, with the cap omitted and the peripherical spring shown in broken lines, illustrating various adjustment possibilities;

FIG. 6 is a plan view of another embodiment of the invention;

FIG. 7 is a section taken on line VII-VII of FIG. 6;

FIG. 8 is a view similar to FIG. 3 but of the embodiment in FIG. 6; and

FIG. 9 is a perspective view of the diaphragm in FIG. 6, shown bent in preparation for installation.

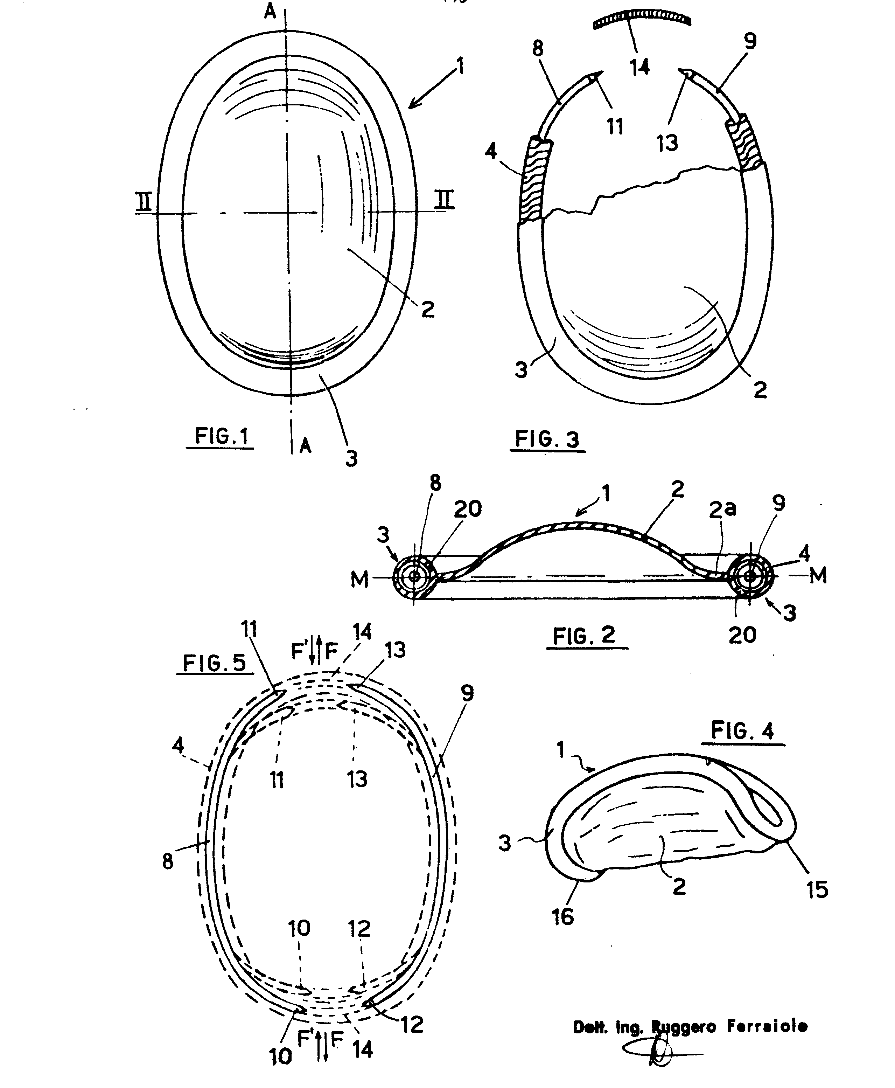

The diaphragm 1 of FIG. 1 consists of the dome 2 made of rubber and of the peripherical structure 3 formed by a spring 4

Dott. Ing. Ruggero Ferraiolo

of circular cross section and by the edge 20 of the dome 2 which is wound, not cast, and conveniently fixed around the spring 4, so forming a sleeve surrounding the spring 4.

Within the spring 4 two bendable shape-retaining steel arched sections 8, 9 of circular cross section are housed, symmetric about axis A-A of FIG. 1, each arched section being provided with pointed ends 10, 11 and 12, 13 which conveniently penetrate two small coil springs 14. The continuity between the sections 8, 9 and the small springs 14 does not prevent the diaphragm from being bent, as shown in FIG. 4, in the area of the small springs 14, in the sections 15, 16.

As illustrated in FIG. 5, the longitudinal dimension of the diaphragm 1 may be varied by some extent. If the central lengths of the arched sections 8, 9 or the lengths close to the ends thereof are conveniently bent outwardly in the direction of arrow F (FIG. 5), the diaphragm extends. On the contrary, if the ends of said lengths are conveniently pressed inwardly in the direction of arrow F', the diaphragm shortens.

The dome 2 is joined via portion 2a to the peripherical structure 3 at the level of the mid-plane referred to as M-M in FIG. 2, rather than extending tangentially thereto as in prior art (see elements 2' and 3' in FIG. 2a).

It will be appreciated that the oval diaphragm provided with bendable shape-retaining arched sections of circular cross section may have the spring 4 and consequently the whole peripherical structure 3 of oval cross section, as an alter-

Dott. Ing. Ruggero Ferraiolo

native to the circular cross section.

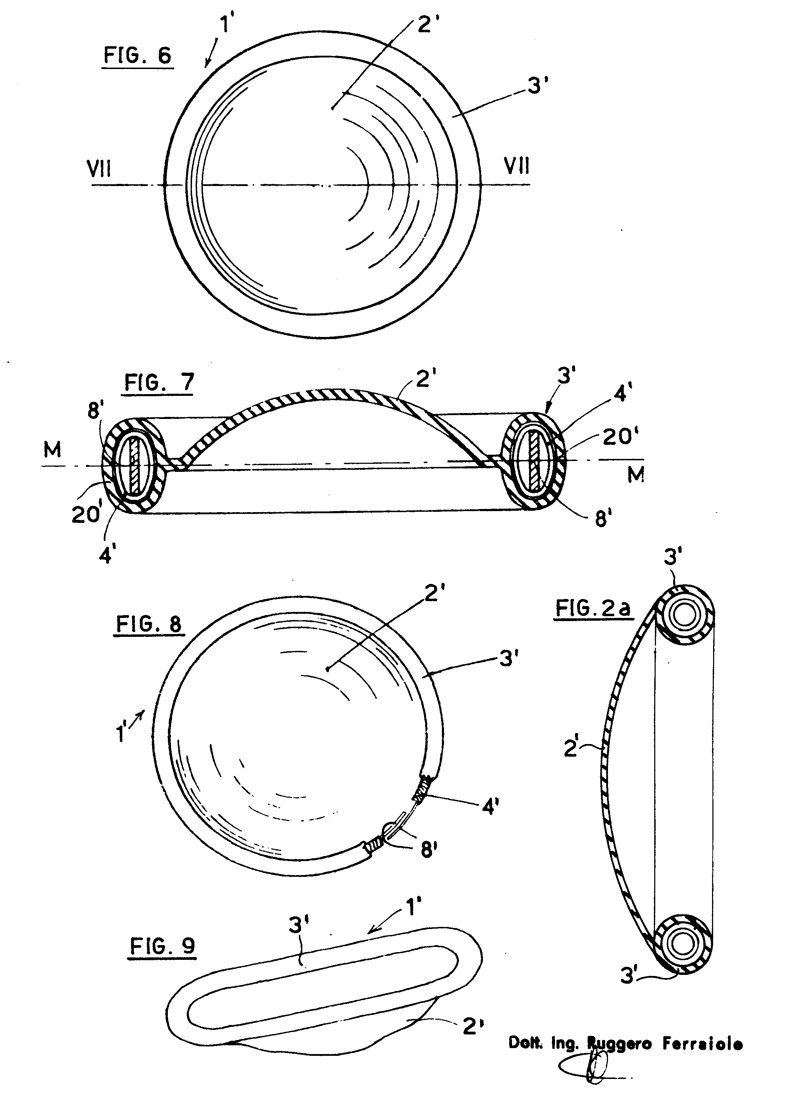

The diphragm 1' of FIG. 6 consists of the dome 2'made of soft rubber and of the peripherical structure 3' formed by a closed spring 4' of oval cross section and by the edge 20' of the dome 2' which is wound, not cast, and conveniently fixed around the spring 4'. Within the spring 4' is housed a bendable shape-retaining steel circular closed section 8' of rectangular cross section; such section 8' is closed peripherically and the ends thereof are overlapped by a few millimeters, as shown in FIG. 8. If the overlap is more than a few millimeters, the overlapped ends may be surrounded by one or more rings (not shown) within the spring 4'. Consequently, if a pull or a compression is exerted radially on the peripherical structure 3', then the diaphragm 1' extends or shortens, respectively, so providing for adjustment according to the specific requirements. Finally, FIG. 9 shows the diaphragm 1' bent for being introduced in the vaginal cavity.

Dott. Ing. Ruggero Ferraiolo

Claims:

1. Vaginal diaphragm consisting of a dome made of elastomeric material which is joined to a peripherical closed coil spring housing a bendable shape-retaining armour characterized in that a peripherical structure (3) of oval shape is straddley by a dome (2) of oval periphery, two bendable shape-retaining arched sections (8, 9) housed in a closed coil spring (4) incorporated in the peripherical structure (3) are bent according to said oval shape, said arched sections (8,9) being such that they may be deformed by the fingers in order to adjust to the specific requirement the length of a selected diaphragm; in that the dome (2) is joined to the inner part of the peripherical structure (3) at the level of the mid-plane (M-M) of the diaphragm (1); in that the cross section of the peripherical structure (3) is substantially circular shaped.

2. Vaginal diaphragm according to claim 1 characterized in that the cross section of the peripherical structure (3) is substantially oval shaped and the major axis of this oval section is perpendicular to said mid-plane (M-M).

3. Vaginal diaphragm consisting of a dome of elastomeric material which is joined to a peripherical closed coil spring housing a bendable shape-retaining armour characterized in that a peripherical structure (3) of circular shape straddled by a dome (2') of circular periphery and incorporating a closed coil spring (4') houses a circular armour consisting of a bendable shape-retaining section (8') of flat cross section the ends of which face each other; in that the dome

Dott. Ing. Ruggero Ferraiole

(2') is joined to the inner part of the peripherical structure (3') at the level of the mid-plane (M-M) of the diphragm (1'); in that the cross section of the peripherical structure (3') is substantially oval shaped and the major axis of this oval section is perpendicular to the mid-plane (M-M) of the diaphragm (1').

4. Vaginal diaphragm according to claim 3 characterized in that the ends of the bendable shape-retaining circular section (8') of flat cross section overlap one another circumfe rentially.

Dott. Ing. Ruggero Ferraiole

- 1/2 -

0006609

FIG.1

FIG. 3

FIG. 2

FIG. 5

FIG. 4

Dott. Ing. Ruggero Ferraiolo

0006609

**FIG. 6**

1'  2'  3'

VII ——— VII

**FIG. 7**

8'  2'  3'  4'

M ——— M

20'  20'  8'

4'

**FIG. 8**

2'  3'

1'

4'

8'

**FIG.2a**

3'

2'

3'

**FIG. 9**

3'  1'

2'

Dott. Ing. Ruggero Ferraiolo

## DOCUMENTS CONSIDERED TO BE RELEVANT

| Category | Citation of document with indication, where appropriate, of relevant passages | Relevant to claim |
|---|---|---|
| X | <u>US – A– 2 529 363</u> (R.J. BALLARD et al.)<br>* column 1, lines 35 to 45;<br>column 3, lines 13 to 15;<br>fig. 2 to 4, positions 12, 13<br>15, 20, 21, 23 *<br><br>-- | 1 |
| X | <u>US – A– 2 638 896</u> (J.T. CLARK)<br>* claim 2; fig. 3, 6, positions 1,<br>3, 4, 5 *<br><br>-- | 3,4 |
| | <u>GB – A – 961 880</u> (ORTHO PHARMACEUTICAL CORP.)<br>* fig. 5 *<br><br>-- | 1,3 |
| | <u>US – A – 3 060 931</u> (J.T. CLARK)<br>* fig. 7, 9, position 2 *<br><br>-- | 1,3 |
| | <u>US – A– 3 169 894</u> (E. MONETT)<br>* fig. 4 *<br><br>-- | 1,3 |
| | <u>US – A – 2 823 669</u> (E. KUNNAS JR.)<br>* claim 1; fig. 1, 2, 4,<br>positions $r'$, $r^2$, 12, 15, 16, 17, 18*<br><br>--<br>./.. | 1 |

### CLASSIFICATION OF THE APPLICATION (Int. Cl.)

A 61 F   5/46

### TECHNICAL FIELDS SEARCHED (Int.Cl.)

A 61 F   5/46

A 61 M   31/00

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | The present search report has been drawn up for all claims | |
|---|---|---|
| Place of search<br>Berlin | Date of completion of the search<br>18-10-1979 | Examiner<br>DROPMANN |

European Patent Office

**EUROPEAN SEARCH REPORT**

0006609

Application number

EP 79 102 122.3

- page 2 -

| Category | Citation of document with indication, where appropriate, of relevant passages | Relevant to claim |
|---|---|---|
| | DE - C - 557 914 (R. KLOTZ)<br>* lines 20 to 21 and 29 to 33;<br>  fig. 1, position a *<br><br>-- | 2,3 |
| A | US - A - 2 463 356 (J.T. CLARK)<br>* column 1, lines 1 to 4; fig. 3 *<br><br>-- | |
| A | US - A - 2 087 610 (R.W. SCOTT)<br>* page 1, lines 7 to 9 and 16 to 20;<br>  fig. 1 *<br><br>-- | |
| A | US - A - 2 875 755 (W.J. HEUBOSKI<br>  et al.)<br>* fig. 18 to 25 *<br><br>-- | |
| A | US - A - 2 443 943 (T. YOUNG)<br>* fig. 2 *<br><br>-- | |
| A | US - A - 2 676 589 (W.J. HEUBOSKI<br>  et al.)<br>* fig. 4 *<br><br>-- | |
| A | US - A - 2 664 082 (W.J. HEUBOSKI<br>  et al.)<br>* fig. 1 *<br><br>---- | |

**DOCUMENTS CONSIDERED TO BE RELEVANT**

CLASSIFICATION OF THE APPLICATION (Int. Cl.3)

TECHNICAL FIELDS SEARCHED (Int. Cl.)